# FASCICULE DE BREVET EUROPEEN

(11) **EP 0 776 651 B1**
(45) Date de publication et mention de la délivrance du brevet: **13.12.2000**
(21) Numéro de dépôt: 96480094.0
(22) Date de dépôt: 23.08.1996
(51) Int. Cl.: A61K 7/06

(54) **Lotion capillaire pour l'arrêt de la chute des cheveux et leur repousse**
Haar-Lotion gegen Haarausfall und zur Förderung des Haarwuchses
Hair lotion to stop hair loss and to induce hair growth

(30) Priorité: 08.12.1995 FR 9514817
(43) Date de publication de la demande: 04.06.1997
(73) Titulaire: Candia, Louis, 06100 Nice (FR); Candia, Marc, 06100 Nice (FR)
(72) Inventeur: Candia, Louis, 06100 Nice (FR); Candia, Marc, 06100 Nice (FR)
(74) Mandataire: Hautier, Jean-Louis

(56) Documents cités:
- BE-A- 486 297
- FR-A- 1 189 821
- GB-A- 2 204 488

## Description

L'invention a pour objet une lotion capillaire pour l'arrêt de la chute des cheveux, et leur repousse.

La lotion capillaire selon l'invention est particulièrement efficace pour soigner le psoriasis.

L'état de la technique peut être défini par les brevets suivants :
- *FR-A-2.598.912 : L'invention a pour objet une composition capillaire pour le traitement des matières kératiniques et en particulier pour la repousse des cheveux, la croissance, l'irrigation du cuir chevelu et la prévention de la chute des cheveux, caractérisée en ce qu'elle comporte les éléments suivants : pétrole, eau, soufre, teinture d'iode, parfum et citron.*
- *FR-A-2.490.960 : L'invention a pour objet un produit assurant la repousse des cheveux et la revitalisation des cheveux blancs comprenant deux lotions que l'on passe l'une après l'autre :*
   *1° Feuilles de romarin macérées dans de l'acoolat de romarin;*
   *2° Feuilles de romarin macérées dans de l'huile de ricin.*
- *EP-0327220 L'invention a pour objet une composition, agent de conservation, à large spectre, comprenant a) un donner de formaldéhyde, et b) un dérivé halogénopropynylique ; des formulations contenant de telles compositions, en particulier des produits de ménage et de soins individuels ; un procédé d'inhibition de la croissance de microorganismes comprenant la mise en contact desdits microorganismes avec la composition d'agent de conservation.*
- *WO-9119562 : L'invention se rapporte à un nouveau composite comprenant un agent tensioactif ampholytique et/ou un agent tensioactif semi-polaire, un acide gras d'ordre supérieur et si nécessaire un minéral à base d'argile ; ainsi qu'à une composition d'émulsion contenant ce composite.*
- *EP-0546235 : L'invention a pour objet un shampooing à partir de constituants naturels pour application sur le cuir chevelu constitué d'un mélange d'huile de ricin, d'amande, d'olive et avantageusement de coco. De préférence, on ajoute également de la glycérine et/ou de l'huile de paraffine.*
- *EP-0498924 : l'invention concerne un produit capillaire à base d'extraits végétaux à activité améliorée, contenant une combinaison des substances suivantes : 1-30 ml d'huile de genévrier en milieu végétal. 2-30 ml d'huile de valériane, 6-10 ml d'huile de bouleau en milieu paraffinique, 6-10 ml d'huile de feuilles de groseillier en milieu végétal, 6-10 ml d'huile capillaire d'arôme de bardane en milieu paraffinique 6-12 ml d'huile capillaire de rose en milieu paraffinique, 2-5 ml d'huile constituée de 90% d'huile d'olive, en milieu végétal, 0,1 -1,2 g de feuilles de menthe poivrée, 0,2-3 amandes douces sans coquille, 0,1-1,2 g de feuille d'ortie, 15-70 grains de jus de radis glacé. 20-80 grains de choux rave, 15-70 graines de trigonelle (foin grec), 15-70 grains de moutarde jaune, 2-8 morceaux de pomme de 1 cm3.*
- *FR-A-2.669.531 : L'invention a pour objet une composition à usage capillaire permettant de dégraisser les cheveux, de supprimer les démangeaisons du cuir chevelu, d'éliminer les pellicules et de réduire et/ou stopper la calvitie, constituée de fleur de soufre, de teinture d'iode, d'essence d'ail, d'huile de paraffine, d'huile* *de cade, d'essence de citron et d'essence de menthe.*
- *GB-A-2 204 488 : L'invention concerne des compositions pharmaceutiques contenant une solution alcoolique d'acide salicylique, d'alun, et d'iode, pour le traitement ou la prévention des affections cutanées, telles que la dermatite, le psoriasis, l'eczéma, et la chute des cheveux.*

La lotion capillaire pour l'arrêt de la chute des cheveux et leur repousse est constituée d'une combinaison des substances suivantes : de teinture d'iode, d'huile de ricin, d'un extrait de feuilles de "lierre" et d'alcool.

La composition précise de ladite lotion capillaire est la suivante, pour 100 g :
- 30 g de teinture d'iode
- 25 g d'huile de ricin
- 5 g d'un extrait de feuilles de "lierre"
- 40 g d'alcool à 70°.

## Revendications

1. Lotion capillaire pour l'arrêt de la chute des cheveux et leur repousse caractérisée par le fait
qu'elle est constituée d'une combinaison des substances suivantes : de teinture d'iode, d'huile de ricin, d'un extrait de feuilles de "lierre" et d'alcool.

2. Lotion capillaire pour l'arrêt de la chute des cheveux et leur repousse, selon la revendication 1 caractérisée par le fait
que la composition de ladite lotion capillaire est la suivante, pour 100 g :
- 30 g de teinture d'iode
- 25 g d'huile de ricin
- 5 g d'un extrait de feuilles de "lierre"
- 40 g d'alcool à 70°.

## Patentansprüche

1. Haar-Lotion gegen Haarausfall und zur Förderung des Haarwuchses, dadurch gekennzeichnet, daß sie aus der Kombination der folgenden Substanzen besteht: Jodtinktur, Rizinusöl, einem Extrakt aus "Efeu"-Blättern und Alkohol.

2. Haar-Lotion gegen Haarausfall und zur Förderung des Haarwuchses gemäß Anspruch 1, dadurch gekennzeichnet, daß die Zusammensetzung der besagten Haar-Lotion wie folgt ist, für 100 g:
- 30 g Jodtinktur
- 25 g Rizinusöl
- 5 g eines Extrakts aus "Efeu"-Blättern
- 40 g Alkohol zu 70 %.

## Claims

1. Hair lotion to stop hair loss and induce hair growth characterised in that it consists of a combination of the following substances:
iodine tincture, castor oil, "ivy" leaf extract and alcohol.

2. Hair lotion to stop hair loss and induce hair growth according to claim 1 characterised in that
the composition of the said hair lotion is as follows, per 100 grams:
- 30 grams of iodine tincture,
- 25 grams of castor oil,
- 5 grams of ivy leaf extract,
- 40 grams of alcohol at 70°.
